# EUROPÄISCHE PATENTSCHRIFT

(11) **EP 1 202 690 B1**
(45) Veröffentlichungstag und Bekanntmachung des Hinweises auf die Patenterteilung: **21.07.2004**
(21) Anmeldenummer: 00958387.3
(22) Anmeldetag: 07.08.2000
(51) Int. Cl.: A61F 5/445

(54) **VORRICHTUNG ZUM AUFFANGEN VON AUS EINEM STOMA AUSTRETENDEN AUSSCHEIDUNGEN MIT EINER VORLAGE**
DEVICE WITH A BAG FOR COLLECTING EXCRETIONS FROM A STOMA
DISPOSITIF DESTINE A COLLECTER DES EXCRETIONS STOMACALES AU MOYEN D'UN RECIPIENT RECEPTEUR

(30) Priorität: 09.08.1999 DE 19936760; 15.10.1999 DE 19949885
(43) Veröffentlichungstag der Anmeldung: 08.05.2002
(73) Patentinhaber: Riesinger, Birgit, 48346 Ostbevern (DE)
(72) Erfinder: Riesinger, Birgit, 48346 Ostbevern (DE)
(74) Vertreter: Hoffmeister, Helmut, Dr. Dipl.-Phys.
(86) Internationale Anmeldenummer: PCT/EP2000/007639
(87) Internationale Veröffentlichungsnummer: WO 2001/010363

(56) Entgegenhaltungen:
- WO-A-98/20821
- US-A- 4 519 797
- US-A- 4 705 512
- US-A- 4 886 509
- US-A- 5 026 362

## Beschreibung

Die Erfindung betrifft eine Vorrichtung zum Auffangen von aus einem Stoma austretenden Ausscheidungen, umfassend eine mit einem Durchlaß versehene Tasche aus flexiblem Material, in welche ein auswechselbarer Sammelteil zum Auffangen der Ausscheidungen einlegbar ist, der um das Stoma herum anliegend anzuordnen ist.

Das Auffangen von Körpersekreten aus natürlichen und nicht-natürlichen Körperöffnungen bei menschlichen Patienten stellt für diese Patienten ein großes Problem dar, wenn durch körperliche Mängel, durch chirurgische oder pathologische Veränderungen oder sonstige Einflüsse die Ausscheidungen einen komplizierten Weg nehmen müssen, der dem Patienten unangenehm ist und der ihn vom gesellschaftlichen Leben in vieler Beziehung ausschließt.

Es sind verschiedene Versorgungssysteme für Stoma-Patienten erhältlich, so daß davon ausgegangen werden kann, daß Anforderungen und Zuverlässigkeit nicht prinzipiell unerfüllt sind. Im wesentlichen sind die bekannten Lösungen darauf gerichtet, die aus einem Stoma austretenden Ausscheidungen in einem Beutel aufzufangen und in einem solchen Beutel zu neutralisieren und mit ihm zu entleeren. Auch wenn bereits vorgeschlagen worden ist, derartige Beutel mit einem flüssigkeitsabsorbierenden Material auszustatten, so bleibt dennoch das Problem, diesen Beutel unauffällig und umweltgerecht zu tragen und zu entsorgen.

So zeigt die US 5 026 362 zeigt eine Stomabeutel-Halterung zur stabilen Anbringung eines herkömmlichen Stomabeutels am Körper des Patienten. Zu diesem Zweck ist ein Gürtel vorgesehen, an dem die Tasche ggf. mit einer Klappe über Klettverschlüsse befestigt ist. Die Vorrichtung weist keine Absorber-Vorlage mit Öffnung auf.

Ferner ist in der US 4 705 512 eine Tasche mit Klappe dargestellt, die zur Aufnahme nur eines Teils eines herkömmlichen Auffangbeutels vorgesehen ist. Durch das Hochschwenken der Klappe kann der ganze an das Stoma angeschlossene Auffangbeutel gleichsam versteckt werden. Sollte der mit den Ausscheidungen gefüllte Auffangbeutel durch die verschwenkte Klappe zusammengedrückt werden, so können die Ausscheidungen zurück in das Stoma fließen. Diese Tasche dient ausschließlich als eine mit der Unterwäsche integrierte Stabilitätsvorrichtung, die dem Patienten ein gewisses Sicherheitsgefühl gewährleisten kann.

Die US 4 886 509 zeigt einen mit Superabsorber versehenen Auffangbeutel, welcher für Urin bestimmt und an natürliche anatomischen Körperöffnungen des an altersbedingte. Blasenschwäche leidenden Patienten anzubringen ist. Nachteilig bei dem genannten Auffangbeutel ist, daß er keine Möglichkeit vorsieht, die Gasaustritte zu kontrollieren und den Superabsorber auszuwechseln. Es besteht keine Anwendungsmöglichkeit bei chirurgisch angelegten Veränderungen.

Der US 4 519 797 ist ein Urostomiebeutel mit einem hautschonenden Überzug zu entnehmen. Der Urostomiebeutel ist mit einem verschließbaren Ablauf und der Überzug mit einer Öffnung zur Entleerung des Beutels versehen. Die Vorrichtung kann nicht als Ileo-, Colo- oder Fistelbeutel verwendet werden.

In der Schrift W098/31402 ist ein Wundpflaster beschrieben, der aus folgenden Teilen besteht: aus einem dampfdurchlässigen Backsheet, aus einer flüssigkeitsabsorbierenden, inneren Vorlage mit Baumwollfasern als Trägerschicht mit darin verteilten Superabsorberteilchen und aus einem direkt auf den Körper des Patienten auflegbaren, netzartigen Topsheet aus Polyurethan. Die Vorlage ist indirekt um die Körperöffnung klebend befestigbar.

Eine weitere Schrift US-A-5 409 472 zeigt ebenfalls einen Wundpflaster, insbesondere einen nachgiebigen Erste-Hilfe-Verband mit einer absorbierenden Schaumschicht und mit unterbrochenen Klebeschichten. Die Dicke der Schaumschicht ist an deren Rändern kleiner als zwischen den Rändern. Der ganze Verband ist feuchtigkeitsdampfdurchlässig.

Schließlich zeigt die Schrift US-A-4 826 497 einen faserigen, geruchsneutralisierenden Artikel, dessen Wasseraufnahmekapazität relativ niedrig ist.

Für eine Erleichterung der den Patienten entstehenden Probleme stellt sich daher die Aufgabe, von einem Auffangbeutel abzugehen und eine sowohl kostenmäßig günstigere als auch für den Patienten akzeptablere und bequemere Vorrichtung vorzuschlagen.

Diese Aufgabe wird bei einer Vorrichtung zum Auffangen und Sammeln von aus einem Stoma austretenden Ausscheidungen, die eine mit einem Durchlaß versehene Tasche aus flexiblem Material umfasst, in welche ein auswechselbarer Sammelteil zum Auffangen der Ausscheidungen einlegbar und um das Stoma herum anliegend anzuordnen ist, dadurch gelöst, dass
- die Tasche durch einen Beutel mit einer verschließbaren Klappe gebildet ist,
- die Klappe bei geöffneter Stellung eine Öffnung bildet, durch die der Sammelteil einlegbar und entnehmbar ist,
- der Sammelteil eine flüssigkeitsabsorbierende Vorlage ist, deren Wandung eine Einlassöffnung aufweist,
- und dass die Tasche über wenigstens ein Anschlüsstück an eine Grundplatte für Stomaträger anschließbar ist.

Eine solche Tasche, die möglichst waschbar und desinfizierbar sein soll, kann mit einem ansprechenden Design hergestellt werden. Sie garantiert einen sicheren Halt und kann die bisher bekannten Stoma-Beutel aus Kunststoff völlig ersetzen.

Während die bekannten Stoma-Beutel nach einer Füllung wegzuwerfen oder zu entleeren sind, erlaubt die genannte Tasche es, durch ihre Öffnung die Vorlage zu entnehmen, eine neue anzubringen und danach die Tasche wieder zu verschließen.

Die Vorlage kann mit einem Topsheet versehen sein, das eine Öffnung besitzt, durch die die Ausscheidungen in die dahinter liegenden Zellstoff-Lagen oder -Teilchen, die mit Superabsorber versetzt und angereichert sind, gelangen können. Damit kann die Vorlage den wäßrigen Anteils der Ausscheidungen absorbieren und die übrigen Ausscheidungen weitgehend dehydrieren. Außerdem können Geruchsinhibitoren und Gasfilter vorhanden sein, mit denen unangenehme Gerüche verhindert werden können.

Um die Vorteile der vorgenannten Lösung zu erläutern, muß man den zu versorgenden Gegenstand erörtern. Neben Stomata, die bei Colostomien, Ileostomien und Urostomien auftreten, sind nässende Öffnungen vorhanden, die schwierig zu versorgen sind und bei denen Heilungsprozesse oft kaum möglich sind.

Körperöffnungen aller vorgenannten Arten können mit der vorgenannten Vorlage, also mit einer Art Inkontinenzvorlage versehen werden. Es kann dabei auf gängige und bereits am Markt etablierte Artikel zurückgegriffen werden, die in neuer Zusammenstellung genutzt werden. Die Verwendung guter und gleichzeitig handelsüblicher Produkte, die im übrigen eine Kassenleistung sind, die in neuer Konstellation auf die patienteneigenen Bedürfnisse ausgerichtet ist, stellt eine wesentlich verbesserte Versorgungsmöglichkeit dar, insbesondere, da nicht nur einige wenige Problemfälle, sondern alle Stomata-Patienten die Vorteile nutzen können.

Geht man vorzugsweise davon aus, daß die Vorlage die Form einer Blase, eines Balges oder eines Säckchens hat, so kann eine solche Vorlage auf einer handelsüblichen Basisplatte, z.B. mit einer benötigen Öffnung, aber vorzugsweise ohne Rastring angeheftet, aufgeklebt oder anderweitig befestigt werden. Die Grundplatte wird entsprechend ihrer normalen Funktion um die zu versorgende Öffnung auf die Haut geklebt. Hautschutz und Haftunterlage sind daher wie üblich gegeben. In Material und Funktionsart entspricht die Vorlage einer Inkontinenzvorlage, wie sie beispielsweise aus der US-Patentschrift 4 886 509 bekannt ist. Die neue Vorlage soll allerdings die Funktion erfüllen, aus dem Körper austretende Ausscheidungen auffangen zu können und deren Flüssigkeitsgehalt zu absorbieren.

Die Vorlage kann an der Grundplatte befestigt sein und sich damit nahtlos und abgedichtet um die Körperöffnung anlegen. Abweichend von der bisher beschriebenen Form kann die Vorlage aus einem mit einem Superabsorber versehene Kissen oder aus einer mit einem Superabsorber versehenen Absorptionslage bestehen.

Die Vorlage kann in Form eines Nestes oder Balges mit einem Einlaß gestaltet sein, der in Höhe des Taschen-Durchlasses angeordnet ist. Die Vorlage kann auch mit einem wasserundurchlässigen, jedoch luftdurchlässigen Backsheet, wie es aus der Windeltechnologie bekannt ist, versehen sein.

Die Tasche wird vorzugsweise aus einer wasch-und/oder desinfizierbaren Folie oder einem entsprechenden Gewebe hergestellt. Vorzugsweise ist die Tasche entsprechend der Volumenzunahme der Absorptionselemente bzw. der flüssigkeitsabsorbierenden Struktur dehnbar.

Dabei wird, um eine Einhand-Bedienung zu ermöglichen, die Platte zum Öffnen der Tasche in der geöffneten Stellung feststellbar gemacht. Die Klappe kann mittels Klettverschluß, wiederverschließbarer Klebenaht, Magnetverschluß oder dgl. verschließbar gemacht werden.

Weiterhin können Vorrichtungen vorgesehen werden, um die Vorlage um den Durchlaß der Tasche herum befestigt anlegbar zu machen.

Damit wird auch eine veränderte Belastung der Grundplatte möglich, so daß eine verlängerte Tragedauer des geklebten Teiles des Versorgungssystems, das heißt der Grundplatte, geschaffen wird und eine veränderte Kostensituation für den Leistungsträger.

Für den Patienten bieten sich viele Vorteile:
- durch Entfallen der Wasserbewegung und durch die kissenförmige Struktur der Vorlage werden sowohl Gluckergeräusche als auch Flatulenzgeräusche vermindert;
- durch Binden der Flüssigkeit werden bakterielle Vermehrung und entsprechende Gerüche unterdrückt; dies in kann noch durch Beifügung von Geruchsinhibitoren zu der Vorlagestruktur verbessert werden;
- die benutzte Vorlagenstruktur wird auf Grund der Wasseraufnahme der Superabsorber gelartig und bewegt sich damit wesentlich weniger als eine flüssige Füllung der Stoma-Beutel;
- die Vorlage kann sehr viel Flüssigkeit aufnehmen und findet trotzdem Platz in der vorbeschriebenen Tasche;
- der Wundbereich trocknet besser ab, so daß auch der Heilungsprozeß verbessert wird;
- der Patient kann auf dem Bauch liegen und auf dem Bauch liegend schlafen;
- es tritt keine unangenehme "Säckchenbildung" auf, da durch entsprechende Gestaltung der flüssigkeitsaufnehmenden Struktur deren Volumen innerhalb der Tasche verteilt ist;
- die Stoma-Platte wird nicht von einer Flüssigkeit unterwandert;
- auch bei einem unkontrollierten Abreißen und beim Wechseln bleibt die Stomavorlage trocken, während ein Beutel aufreißen und seinen Inhalt ergießen kann;
- die Stomata sind nicht ständig von ätzender Flüssigkeit umspült; trotzdem bleibt der Darm ununterbrochen in dem für ihn wichtigen feuchten Milieu;
- der Patient ist nicht von der Nähe einer Toilette abhängig, da das Wechseln der Vorlage und das weitere Beisichtragen in einem Abfallbeutel möglich sind;
- da die Tasche für viele Vorlagen nacheinander verwendbar ist, ergibt sich hier auch ein wesentlicher Verbilligungseffekt.

Die Erfindung ergibt damit ein Versorgungskonzept für nahezu alle nicht-natürlichen nässenden Körperöffnungen. Es werden nicht nur die Belange der Patienten maßgeblich berücksichtigt, sondern auch medizinische Erfordernisse. Auch die Kostenersparnisse für die Leistungsträger stellen eine Bereicherung für die Solidargemeinschaft dar.

Die Erfindung soll auch die Möglichkeit umfassen, verschiedene Anwendungsmöglichkeiten an die bekannte Stoma-Platte vorzusehen, also klettende, klebende oder angeheftete.

Die Vorlage ist von einer Tasche umgeben, die aus Gründen der Halterung, zur Unterstützung der Tragesicherheit und zum Wechsel der Stomavorlage eingesetzt werden kann. Die Tasche soll waschbar und desinfizierbaren sein und ebenfalls an der Stoma-Platte anzubringen sein, beispielsweise durch eine Rastvorrichtung.

Die Tasche soll auch die Möglichkeit bieten, mit einem Bauchgurt getragen werden zu können, so daß die Tasche, die mit Riemen, Riemenösen alle dgl. Vorrichtungen versehen sein kann, im Abstand von der Stomaöffnung befestigbar ist. Darüber hinaus kann die Tasche auch mit einer Anschlußleitung für eine Schlauchleitung versehen sein, die es ermöglicht, wiederum mit einer Abflußmöglichkeit im Inneren der Tasche verbunden zu werden, so daß eventuell unerwünschte Flüssigkeiten abgelassen werden können.

Die Tasche und/oder die Vorlage kann mit einem Digestionsgase oder Fäulnisgase absorbierenden Filter versehen sein, der vorzugsweise mit der Klappe zum Öffnen der Tasche abnehmbar verbunden ist. Der Filter kann aber auch frei in der Tasche plaziert sein.

Wegen der langen Lebensdauer der Tasche ist es auch möglich, diese direkt mit einer Stomaplatte werksseitig zu verbinden.

Mit der Verwendung der Tasche soll erreicht werden, daß das Tragen und Wechseln der Vorlage erleichtert sind, das gebundene Ausscheidungen in einer Vorlage gesammelt werden, wobei die Vorlage über eine Öffnung in der Tasche entnommen werden kann und durch eine neue Vorlage ersetzbar ist. Darüber hinaus soll eine dreifache Tragesicherheit gewährleistet sein, nämlich durch die Verbindung direkt an der Platte, an der die Vorlage eingebracht wird. Ein zielsicherer Halt wird durch die Tasche gewährt, die die Stomavorlage trägt, sobald sie durch deren Gewicht bedingt von der Platte abfallen sollte; darüber hinaus wird ein Bauchgurt vorgesehen, der die Tasche hindert, mit der Vorlage von der Basisplatte abzufallen, wenn sich die Befestigung an der Platte insgesamt lösen sollte.

Man hat damit eine Vorrichtung zum Auffangen von körpereigenen Ausscheidungen aus natürlichen und nicht-natürlichen Körperöffnungen, bestehend aus,
- einem sackförmigen, eckigen, rundlichen, beutelförmigen oder anders geformten Sammelteil in Form einer saugenden Inkontinenzvorlage gängigen Materials, gegebenenfalls mit Abweichungen, zum Auffangen der oben genannten Ausscheidungen und zum Absorbieren oder Binden des wäßrigen Anteils der Ausscheidungen; dieses Sammelteil ist in Form einer flexiblen, gegebenenfalls saugenden, untypisch geformten Stomainkontinenzvorlage um die Körperöffnung herum anlegbar und über unterschiedliche Mechanismen im Raum der Körperöffnung selbst anzubringen,
- einer die oben bezeichnete Stomainkontinenzvorlage umgebenden Tasche aus flexiblem Material, gegebenenfalls wasch-, abnehm- und desinfizierbar, die mit einem Anschluß und einem Durchlaß direkt oder indirekt an die Körperöffnung versehen ist, und die es erlaubt, über eine verschiedentlich geformte und verschiedentlich zu öffnende und/oder zu verschließende fensterartige Öffnung die Stomainkontinenzvorlage zu entnehmen, eine neue einzugeben und die Fensteröffnung selbst zu verschließen, wobei die Vorlage selbst und die Tasche gegebenenfalls mit unterschiedlichen, medizinisch oder verbraucherbedingt sinnvollen Materialien beschichtet sein kann und das Fenster nicht klarsichtig sein muß,
- einer Zusammenstellung der oben aufgeführten Artikel, derart, daß die oben auch Sammelteil genannte Stomainkontinenzvorlage innen an dem Durchlaß der Tasche zum Auffangen und sicheren Binden der Ausscheidungen anliegt, wobei gegebenenfalls die Tasche und/oder die Vorlage mit einem Filter ausgestattet werden können, der als eigenständiges Sonderteil oder eingebaut in einen Teil der zu bezeichnenden Vorrichtung seine Funktion aufnehmen kann; denkbar wäre auch der Einsatz eines wasserabsorbierenden Superabsorbers, der auch die Aufnahme und/oder Filterung der durch Gasentwicklung im Darm austretenden unangenehmen Gerüche gewährleistet,
- einer Verbindungsstelle, die die Körperöffnung direkt oder indirekt, d.h. die umgebende Haut direkt oder indirekt die umgebende über z.B. eine angebrachte Haftplatte gängiger Bauart, mit der Tasche verbindet; lose Verbindungen, geklebte, geheftete, geklipste, gerastete, magnetische oder andere medizinisch vertretbare Verbindungsmöglichkeiten sind denkbar,
- einer Anschlußmöglichkeit im oder am Sammelteil für ableitende Inkontinenzartikel, z.B. Katheter, Beinund Bettbeutel, mit dem Ziel, das hier darzustellende Produkt auch für z.B. Katheterträger oder Kondompatienten nutzbar zu machen,
- einer Anschlußmöglichkeit an die zu versorgende Körperöffnung z.B. in Form einer handelsüblichen Basisplatte, die z.B. bei Stomaträgern als Grundplatte für die Anschließbarkeit des Versorgungssystems genutzt wird, um die für das hier vorzustellende Versorgungssystem derartig bearbeitet wurde, daß die Tasche und auch die Stomainkontinenzvorlage daran anklebbar, klipsbar, rastbar, heftbar, magnetisch verbindbar oder anders zusammenhaltend zufügbar ist;
- einem Sammelteil in Form der in Anspruch 1 dargestellten Stomainkontinenzvorlage mit wählbaren Folienqualitäten und eingegebenen Absorbermengen, die sich darstellen können vor absorberfreien Vorlagen bis zu überdimensioniert großen Mengen an Absorber in der Vorlage; dieser Sammelteil ist nicht oder, je nach Einsatzgebiet, innen oder außen mit verbraucherorientiert wirksamen Materialien versehen, die wäßrige Stoffe, Gerüche oder Schallentwicklungen aufnehmen oder dämpfen; dieses Sammelteil kann wahlweise geöffnet werden, an nicht bestimmter Stelle, um zusätzlich ein absorbierendes Zusatzelement zuzugeben oder zu entnehmen, wobei dieses Sammelteil direkt oder ein haftendes Zusatzteil an die Körperöffnung versorgungstechnisch vertretbar anzulegen ist.

Die Tasche kann mit einer zu öffnenden oder zu verschließenden Öffnung versehen sein, durch die der Sammelteil entnehmbar und einlegbar ist und in deren Verschluß gegebenenfalls ein weiterer Filter eingebaut sein kann.

Der Sammelteil kann wenigstens ein mit einem Superabsorber wählbarer Qualität versehenes Kissen, Sachet oder anderes saugendes Vlies verschiedener Form umfassen.

Die Stomainkontinenzvorlage, d.h. der Sammelteil, kann in Form eines weitgehend verschlossenen Nestes mit einem Einlaß gestaltet sein, der in Höhe des Durchlasses der Tasche angeordnet ist und den Eingang in die Vorlage für die Ausscheidungen darstellt.

Die Vorlage kann mit einem wasserundurchlässigen Backsheet versehen sein, Luft aber, je nach Produktionsart und Einsatzgebiet, gegebenenfalls gereinigt entweichen läßt, mit sammelt und nicht durchläßt, um der filternden Funktion gerecht werden zu können.

Die Tasche kann aus einer Kunststoff-Folie, einem Naturstoffgewebe oder aus einem teil-künstlichen Material bestehen, wobei auch "Goretex"-Materialien möglich sein können, damit auch die Desinfizierbarkeit und Waschbarkeit, gegebenenfalls auch in der Waschmaschine, gewährleistet bleiben.

Die Stomainkontinenzvorlage und die Tasche selbst können entsprechende der Volumenzunahme durch Aufnahme und Absorption wäßriger Bestandteile dehnbar und füllbar sein.

Die Tasche kann mit einer abwinkelbaren Klappe, einer wiederverschließbaren Öffnung oder einem anderweitig konstruierten Einlaß versehen sein, die vorzugsweise aus dem Material der Tasche selbst bestehen und den Zugang zu dem auffangenden Sammelteil darstellen; auch Laschen für den Einsatz eines Bauchgurtes zur zusätzlichen Stabilisation können befestigt werden.

Die Klappe kann z.B. mittels Klettverschluß, wiederverschließbarer Schweißnaht, Magnetverschluß oder anderen Anschlüssen mit den anderen Bestandteilen der Tasche verbindbar und verschließbar sein.

Die Tasche kann außen um den Einlaß der Vorlage herum anlegbar sein bzw. die Vorlage teils oder vollständig umkleiden.

Der Sammelteil kann mittels Release-Kleber oder Klettverschluß im Inneren der Tasche befestigt werden.

Die Größe, Form, Absorptionsstärke und Qualität der Vorrichtung mit und ohne Tasche kann derart sein, daß sie sich durch die saugende Vorlage für den Einsatz als Versorgung nässender Öffnungen, d.h. Fisteln, Wunden, Colostomien, Urostomien, Ileostomieen, prae- und postoperativen Narben und Wunden eignet.

Die Tasche kann in ihrer Funktion die Tragestabilität eines Versorgungssystems, die Geruchsneutralität in der Umgebung des Patienten oder die Schall-Leitung von z.B. Flatulenzgeräuschen positiv zum Vorteil des Betroffenen beeinflussen.

Das gegebenenfalls innen nicht überdimensioniert beschichtete Sammelteil kann wahlweise an nicht bestimmter Stelle und in nicht bestimmter Form geöffnet werden, um ein absorbierendes Zusatzelement zuzugeben oder zu entnehmen, wobei dieses Sammelteil direkt über ein haftendes Zusatzteil an die Körperöffnung oder deren Umgebung versorgungstechnisch vertretbar anzulegen ist.

Die Tasche kann gegebenenfalls direkt und ab Werk an die haftende Basisplatte oder an eine andere an die Körperoberfläche haftende Vorrichtung fest angebracht werden und muß daher mit der Basisplatte gewechselt werden. Die auszuwechselnden Stammteile können nach Gebrauch über die oben beschriebene Öffnung in der Tasche allerdings auch entnehmbar und einfügbar sein, so daß ein Versorgungssystem angeboten werden kann, das als fest konstruiertes einteiliges System mit einer Einlaßmöglichkeit für den Wegwerfartikel, den das Sammelteil darstellt, erhältlich ist.

In der Zeichnung sind Ausführungsbeispiele der Erfindung dargestellt. Die Figuren zeigen im einzelnen:
- Fig. 1: eine Stomaöffnung mit Vorlage und geöffnet dargestellter Tasche;
- Fig. 2: die Tasche in geschlossener Darstellung;
- Fig. 3: eine Draufsicht auf die Grundplatte;
- Fig. 4: eine Grundplatte mit einer Vorlage, ohne Tasche;
- Fig. 5: schrittweiser Aufbau der Vorrichtung;
- Fig. 6: eine geänderte Ausführungsform;
- Fig. 7: einen Schnitt durch die Vorrichtung, von der Seite gesehen;

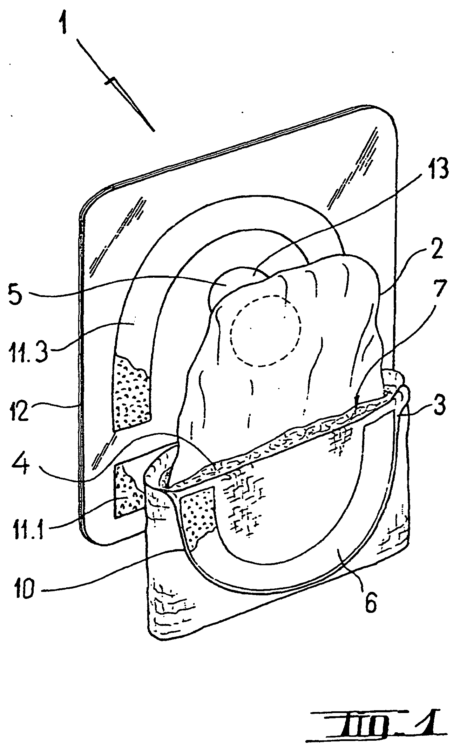

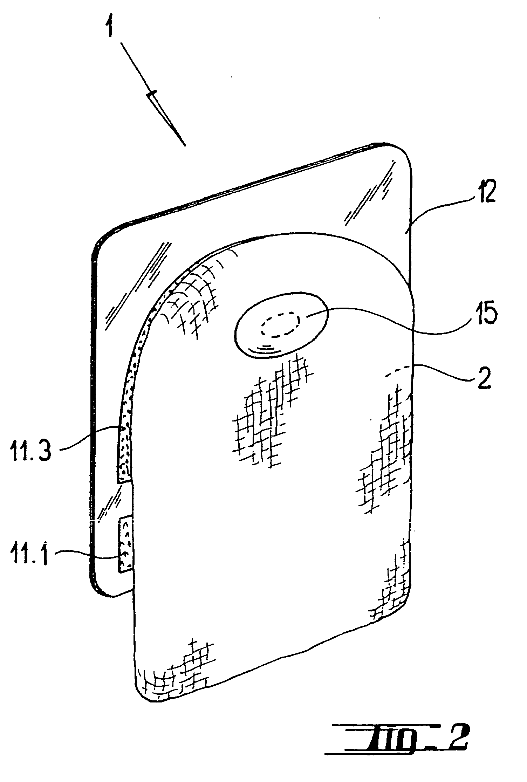

Die in den Figuren 1 bis 3 dargestellte Vorrichtung 1 dient zum Auffangen von aus einem Stoma 13 austretenden Ausscheidungen 4. Die Vorrichtung besteht aus einem Sammelteil in Form einer flexiblen Vorlage 2 und einer mit einer Grundplatte 12 verbundenen Tasche 3 aus flexiblen Kunststoffmaterial, das sowohl gewaschen als auch desinfiziert werden kann.

Die Tasche ist mit einem Anschlußstück 6 in Form eines Klettbandes versehen, das auf der Innenseite einer Klappe 10 angebracht ist, und mit einem entsprechenden, halbmondförmigen Klettverschluß 11.3 auf der Grundplatte verbindbar ist. Die Tasche 3 ist in ihrem unten hängenden Bereich als geschlossener Beutel gestaltet. Sie trägt auf ihrer zu einer Grundplatte 12 des Stoma-Anschlusses zugewandten Seite einen weiteren Klettverschluß, so daß sie an der Grundplatte 12, die ein kompatibles Klettverschlußband 11.1 aufweist, aufgehängt werden kann.

Die Klappe 10 kann nach oben geklappt werden und gegen den entsprechenden Gegenverschluß 11.3 auf der Grundplatte 12 fest angedrückt werden, so daß ein praktisch geruchsdichter Verschluß geschaffen ist.

Wenn die Tasche an der Grundplatte 12 festgelegt ist, wird bei geöffneter Klappe 10 eine Vorlage 2, die zum Auffangen der aus dem Stoma austretenden Ausscheidungen vorgesehen ist, in die Tasche eingelegt, und ebenfalls, vorzugsweise über eine das Stoma völlig umschließende ringförmiger Klebeschicht (nicht dargestellt) an der Grundplatte befestigt.

Die Gestalt der Vorlage ist die eines geschlossenen Säckchens mit einer Öffnung, die dem Stoma 13 zugewandt ist. Die Vorlage 2 besteht, wie an sich bei Kinderwindeln bekannt, aus einem Topsheet, das der Taschenöffnung zugewandt ist und eine Öffnung 7 besitzt, dahinter liegen Zellstoff-Lagen, die mit Superabsorber versetzt und angereichert sind. Damit kann die Vorlage 2 den wäßrigen Anteils der Ausscheidungen absorbieren und die übrigen Ausscheidungen weitgehend dehydrieren. Außerdem können Geruchsinhibitoren und Gasfilter 15 vorhanden sein, mit denen unangenehme Gerüche verhindert werden können.

Weiterhin kann mit einem Riemen, der in entsprechenden Riemenösen geführt ist, die Tasche mit Vorlage weitgehend beschwerdefrei am Körper getragen werden.

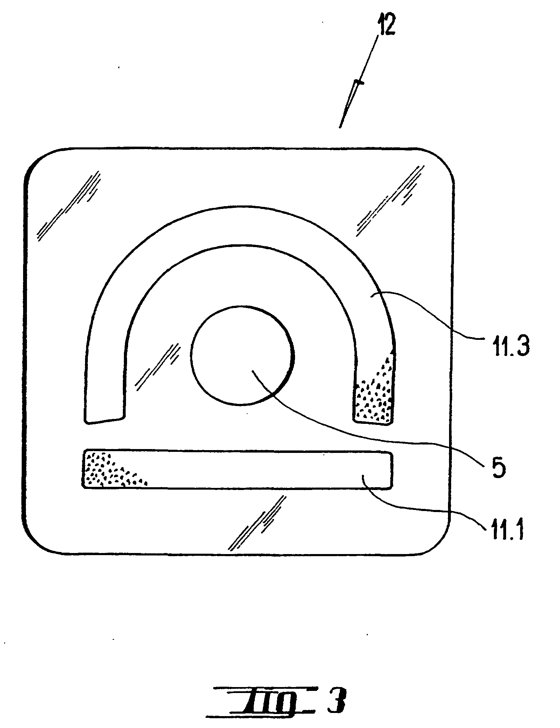

Fig. 3 zeigt eine Grundplatte 12 in Draufsicht. Mittig ist eine Durchlaßöffnung 5, die unmittelbar über dem Stoma zu liegen kommt, zu erkennen. Darüber liegt halbmondförmig ein Klettverschluß-Streifen 11.3 und darunter ein rechteckiger weiterer Klettverschluß-Streifen 11.1. Das Format der Grundplatte 12 ist etwa quadratisch mit einer Seitenlänge von 10 cm.

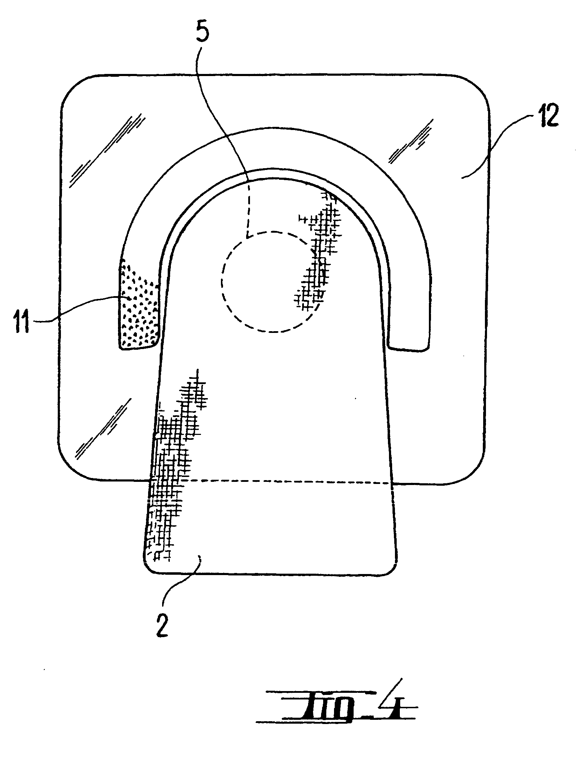

Fig. 4 zeigt die Basisplatte gemäß Fig. 3 mit einer um den Durchlaß 5 eingesetzten Vorlage 2. Es handelt sich hierbei um ein Säckchen aus Polyethylen-Vlies, in das eine thermoplast-gebundene Wirrfaser-Anordnung mit 25 Gew.-% Superabsorbentien eingefüllt ist. Die an sich aus der Windel-Technologie bekannten Superabsorbentien können Wasser bis zum 100fachen ihres Anfangsvolumens aufnehmen. Den in die Vorlage eintretenden Ausscheidungen wird daher das Wasser sofort entzogen. Die Vorlage dehnt sich und füllt nach und nach einem immer größeren Teil der Tasche 3.

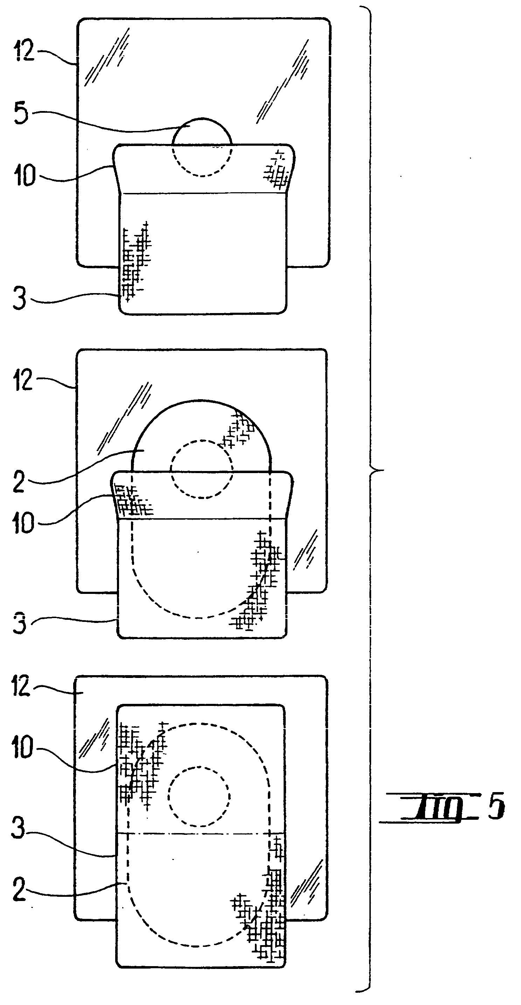

In Fig. 5 sind die Phasen der Stoma-Versorgung dargestellt. Zunächst wird die Tasche 3 an der Grundplatte befestigt, was mit Hilfe des unteren Klettverschluß-Streifens 11.1 geschieht. Anschließend wird die Vorlage 2 bei geöffnete Klappe 10 in die Tasche 3 eingesetzt und über eine Klettverbindung an der Grundplatte befestigt. Anschließend wird die Tasche geschlossen, indem der innen liegende Klettverschluß-Teil der Klappe an den halbmondförmigen Verschlußteil 11 angedrückt wird. Ist die Vorlage 2 verbraucht, so wird sie bei geöffneter Klappe 10 entnommen und durch eine neue Vorlage ersetzt.

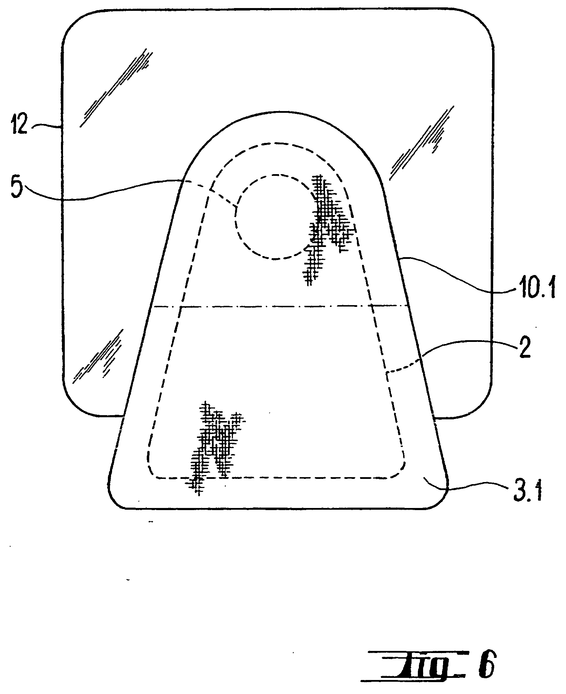

Fig. 6 zeigt eine Ausführungsform einer Tasche 3.1, die etwa eine Dreiecksform hat und in ihrem Inneren eine Vorlage 2 aufnimmt. Die Tasche 3.1 ist im oberen Bereich mit einer Klappe 10.1 versehen, mit deren Hilfe aus der und in die Tasche, wie bereits beschrieben, eine Vorlage einsetzbar und herausnehmbar ist.

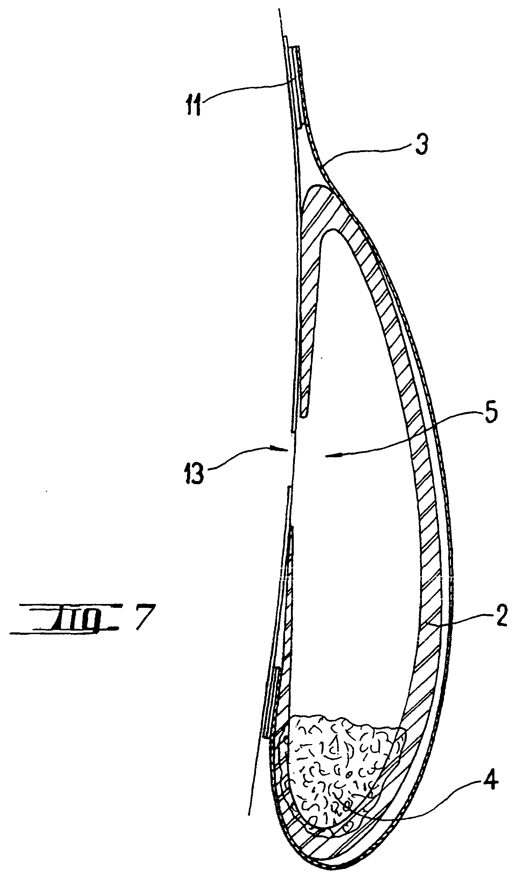

Fig. 7 zeigt eine Vorrichtung gemäß Erfindung im Schnitt bei seitlicher Ansicht. Auf der an die Haut aufgeklebten Grundplatte 12, die das Stoma 13 umschließt, ist über einen Klettverschluß die Tasche 3 hängend befestigt. In ihr befindet sich, quasi in Form eines Nestes, die Vorlage 2. Sie nimmt in ihrem unteren Bereich Ausscheidungen 4 auf, die von dem in der Vorlage vorhandenen Superabsorber dehydriert werden. Im teilgefüllten Zustand hat die Vorlage 2 von der Innenwand der Tasche einen gewissen Abstand, der sich im Laufe der Füllung der Vorlage verringert.

Die Vorlage Könnte aus einer semipermeablen Polyethylenfolie bestehen, die beutelartig geformt ist und mit einer Mischung aus 75 Gew.-% Cellulose-Fasern und 25 Gew.-% Superabsorber gefüllt ist.

Die Polyethylenfolie ist an der Außenseite mit einer kreisringförmigen, zunächst mit einem Releasepapier abgetretenen Klebeschicht (nicht dargestellt) versehen. Nach Abziehen des Releasepapiers wird die Vorlage auf die Grundplatte 12 aufgedrückt und damit angeklebt.

## Patentansprüche

1. Vorrichtung zum Auffangen und Sammeln von aus einem Stoma austretenden Ausscheidungen, umfassend eine mit einem Durchlaß versehene Tasche (3) aus flexiblem Material, in welche ein auswechselbarer Sammelteil zum Auffangen der Ausscheidungen einlegbar ist, der um das Stoma herum anliegend anzuordnen ist,
**dadurch gekennzeichnet, dass**
- die Tasche (3) durch einen Beutel mit einer verschließbaren Klappe (10) gebildet ist,
- die Klappe (10) bei geöffneter Stellung eine Öffnung (7) bildet, durch die das Sammelteil einlegbar und entnehmbar ist,
- der Sammelteil eine flüssigkeitsabsorbierende Vorlage ist, deren Wandung eine Einlassöffnung (5) aufweist,
- und dass die Tasche (3) über wenigstens ein Anschlußstück (11; 11.1, 11.3) an eine Grundplatte (12) für Stomaträger anschließbar ist.

2. Vorrichtung nach Anspruch 1, **dadurch gekennzeichnet, dass** das Anschlußstück (11; 11.1, 11.3) in Form von Klettverschlüssen, wiederverschließbaren Klebenähten, Magnetverschlüssen oder dgl. vorliegt.

3. Vorrichtung nach Anspruch 1 und 2, **dadurch gekennzeichnet, dass** die Tasche (3) entsprechend der Volumenzunahme der Vorlage (2) dehnbar ist.

4. Vorrichtung nach Ansprüchen 1 bis 3, **dadurch gekennzeichnet, dass** die Vorlage (2) mittels Release-Kleber oder Klettverschluß an der Innenseite der Tasche (3) befestigbar ist.

5. Vorrichtung nach Anspruch 2, **dadurch gekennzeichnet, dass** der Beutel beim Abziehen der Klappe (10) von der Grundplatte (12) mit der Grundplatte (12) lösbar verbunden bleibt.

## Claims

1. A device for trapping and collecting secretions from a stoma, comprising a pocket (3) of flexible material which is provided with a passage and into which can be inserted an interchangeable collecting part to trap secretions and which is arranged to be placed around the stoma in abutting relationship, **characterised in that**
- the pocket (3) is formed by a bag with a closable flap (10),
- in the open position the flap (10) forms an opening (7) through which the collecting part can be inserted and removed,
- the collecting part is a liquid-absorbing receptacle, the walls of which have an inlet opening (5),
- and **in that** the pocket (3) is adapted to be connected via at least one connecting member (11; 11.1, 11.3) to a baseplate (12) for stoma carriers.

2. A device according to claim 1, **characterised in that** the connecting member (11; 11.1, 11.3) is in the form of hook and loop fasteners, reclosable adhesive seams, magnetic fasteners or the like.

3. A device according to claim 1 and 2, **characterised in that** the pocket (3) is expandable to correspond to the increase in volume of the receptacle (2).

4. A device according to claims 1 to 3, **characterised in that** the receptacle (2) is adapted to be fixed to the inside of the pocket (3) by means of release adhesives or hook and loop fastening.

5. A device according to claim 2, **characterised in that** when the flap (10) is removed from the baseplate (12) the bag remains releasably connected to the baseplate (12).

## Revendications

1. Dispositif de recueil et de collecte d'excrétions sortant d'une ouverture, qui comprend une poche (3) en matière flexible comportant un passage, dans laquelle peut être inséré un élément collecteur interchangeable de recueil des excrétions qui doit être disposé de manière à s'appliquer autour de l'ouverture,
**caractérisé en ce que**:
- la poche (3) consiste en un sachet à clapet (10) susceptible d'être fermé,
- le clapet (10) forme, en position ouverte, un orifice (7) à travers lequel l'élément collecteur peut être inséré et enlevé,
- l'élément collecteur est un récipient absorbant les liquides, dont la paroi comporte un orifice d'entrée (5), et **en ce que**
- la poche (3) peut être raccordée au moyen d'au moins un élément de raccord (11; 11.1, 11.3) à une plaque de base (12) pour support d'ouverture.

2. Dispositif selon la revendication 1, **caractérisé en ce que** l'élément de raccord (11; 11.1, 11.3) consiste en fermetures de type Velcro, en joints adhésifs susceptibles d'être refermés, en fermetures magnétiques ou similaires.

3. Dispositif selon la revendication 1 ou 2, **caractérisé en ce que** la poche (3) est extensible en fonction de l'augmentation de volume du récipient (2).

4. Dispositif selon l'une quelconque des revendications précédentes, **caractérisé en ce que** le récipient (2) peut être fixé sur le côté interne de la poche (3) à l'aide d'un adhésif détachable ou d'une fermeture du type Velcro.

5. Dispositif selon la revendication 2, **caractérisé en ce que** le sachet reste assemblé de façon détachable à la plaque de base (12) lorsque le clapet (10) est retiré de la plaque de base (12).
